# EUROPEAN PATENT SPECIFICATION

(11) **EP 4 346 561 B1**
(45) Date of publication and mention of the grant of the patent: **18.12.2024**
(21) Application number: 22736357.9
(22) Date of filing: 27.05.2022
(51) Int. Cl.: A61B 5/00, A61B 5/0533, G16H 20/70, G16H 40/63, G16H 40/67

(54) **SYSTEM AND METHOD FOR ASSISTING THE CONDUCT OF ACTIVITIES**
SYSTEM UND VERFAHREN ZUR UNTERSTÜTZUNG DER DURCHFÜHRUNG VON AKTIVITÄTEN
SYSTÈME ET PROCÉDÉ DESTINÉ À L'AIDE À LA CONDUITE D'ACTIVITÉS

(30) Priority: 28.05.2021 IT 202100013985
(43) Date of publication of application: 10.04.2024
(73) Proprietor: Politecnico di Milano, 20133 Milano (IT)
(72) Inventor: SUSCA, Davide, 20133 Milano (IT); BIANCHINI, Massimo, 20133 Milano (IT); MAFFEI, Stefano, 20133 Milano (IT)
(74) Representative: Caruti, Filippo
(86) International application number: PCT/IB2022/055010
(87) International publication number: WO 2022/249142

(56) References cited:
- US-A1- 2005 086 082
- US-A1- 2008 074 951
- US-A1- 2011 224 521
- US-A1- 2019 328 227
- US-A1- 2020 242 952
- US-B1- 9 122 430

## Description

### TECHNICAL FIELD

The present invention refers to the field of automated assistance systems. In particular, the present invention concerns a system for assisting the conduct of activities programmed for a user, e.g. a user suffering from an autism spectrum disorder.

### BACKGROUND

Autism is a neurological condition that persists throughout an individual's lifetime. From the moment of diagnosis in childhood (which usually occurs around the second year of life), autism directly alters the autistic individual's relationship with the environment.

The person's perception of the world becomes fraught with obstacles, physical and emotional barriers that prevent the individual from embarking on a neurotypical life - i.e., similar to the life of a non-autistic person. In order to maintain states that are in respect of autistic sensitivity, typically the sensory and social stimulations that the autistic individual receives on a daily basis are reduced.

However, during adolescence first and adulthood later, the need for social interactions and travel make it impossible to limit sensory and social stimulations. In fact, the person goes through a phase known for its social detachment from the family unit - hence from known and controlled situations and environments - in search of greater economic and personal autonomy. Thus, the autistic individual finds himself in the position of having to learn to manage a number of routine activities autonomously - for example, while being exposed to stimulations that can lead to highly disturbing events, typically referred to as 'meltdowns'.

In an attempt to contain the effect of meltdowns, US 2015/073309 proposes a band provided with accelerometers and configured to identify repetitive movements, referred to as stereotypical movements (hand movements, body rocking, etc.), typically performed by an autistic user during a meltdown event. When repetitive movements are detected, it is provided for a procedure aimed at alleviating meltdown effects, for example the reproduction of a guided meditation or breathing, a song, etc., to be started on a user device - for example a smartphone.

Similarly, US 2015/258302 proposes a wearable device configured to detect repetitive movements of the user or an accelerated heartbeat and to produce a vibration that should attenuate the meltdown. In addition, the wearable device comprises a button whose pressure sends a request for help to a remote-control device held by a guardian or a social health operator.

US 2019/328227 describes a personal management device including a programmable scheduler to enable scheduling activities of a user over a predetermined period. A time-counting function provides alerts for each scheduled activity at the appropriate time. The device envisages monitoring biological indicators and when threshold levels indicative of the onset of anxiety are reached, it interrupts the programmed activity to implement an anxiety-reducing strategy.

US 2005/086082 describes a health care system. The system comprises a portable device. Through the portable device, the patient is reminded when to carry out the activities in the prescribed treatment regimen, and information is collected on the effective patient's compliance with the prescribed treatment regimen and symptoms.

The Applicant has found that the known systems are unable to assist the user in an autonomous management of daily life, which is necessary to ensure that an autistic individual achieves at least partial independence from guardians and social health operators.

### OBJECTS AND SUMMARY OF THE INVENTION

An object of the present invention is to overcome the drawbacks of the prior art.

In particular, it is an object of the present invention to provide a system capable of assisting a user with special needs, such as a user suffering from an autism spectrum disorder, in the execution of one or more programmed activities by developing at least partial independence from guardians and/or social health operators.

A further object of the present invention is to propose a system capable of identifying, preferably in real time, whether and which activities are a source of stress for the user.

In detail, it is the object of the present invention to propose a system that makes it possible to identify vexing events experienced by the user in a simple and effective way by limiting the need for sensors.

These and other objects of the present invention are achieved by a system incorporating the features of the annexed claims, which form an integral part of the present description.

According to a first aspect, the present invention is directed to a system for assisting the z r execution of programmed activities. The system comprises:
a wearable electronic device comprising a sound reproduction module and an interactive portion configured to detect a touch of the user, and
a software application running on a remote terminal suitable for exchanging data with the wearable electronic device.

Typically, the wearable electronic device is worn by a user with special needs, while the software application is run on a remote terminal used by a caregiver of the user - e.g. a relative, guardian, specialised personnel, etc.

The software application comprises an agenda that stores at least one voice message, said voice message comprises an invitation to carry out an activity and is associated with a time instant. In detail, the software application is configured to transmit the at least one voice message and the time instant associated therewith to the wearable electronic device. In turn, the wearable electronic device is configured to reproduce the at least one voice message at the time instant associated with the at least one voice message. Advantageously, the wearable electronic device is configured to detect at least one touch of the interactive portion following the reproduction of the at least one voice message, measure a duration of the at least one touch, and transmit to the software application a number of detected touches and the measured duration of each detected touch. The software application identifies a vexing event experienced by the user when it is detected at least one between a touch lasting longer than a threshold duration value, and a sequence of consecutive touches within a predetermined time interval.

Thanks to this solution, it is possible to indirectly assist the user in the execution of daily activities. Specifically, the system provides the user with the necessary stimulation to carry out one or more activities, e.g. daily activities such as having breakfast, washing, getting dressed, going to a certain place, etc. Through the system, the user thus learns to perform a routine of operations, for example on a daily basis. As a result, the user can achieve an increasing degree of autonomy in the execution of various activities, particularly in the case of activities that have to be repeated daily.

Furthermore, the recording and the analysis of interactions with the interactive portion performed by the system makes it possible to identify the occurrence of stressful situations of the user in relation to the performance of daily activities without the need to resort to complex devices and/or require the acquisition of detailed information about the user or the user's environment. This helps the caregiver to better understand which activities create troubles and/or encounter a decision-making barrier, i.e. they are not actively welcomed by the user (e.g. due to preferences of the user, who might not like doing a certain activity). The identification of these vexing events makes it possible to implement various strategies to address and overcome the trouble itself, for example: exercises aimed at improving the relationship of the user with the specific activity, the subdivision of the activity into shorter and more easily manageable activities or activities in order to be successful in the activity autonomously through a gradual path.

In one embodiment, the agenda stores at least one further voice message containing an instruction to be followed to complete said activity. In this case, the software application is configured to transmit said at least one further voice message to the electronic wearable device. In addition, the wearable electronic device is configured to reproduce said at least one further voice message when a touch of duration equal to or shorter than said threshold duration is detected after the reproduction of the at least one voice message.

In one embodiment, the wearable electronic device is configured not to reproduce said at least one further voice message when a touch lasting longer than said threshold duration is detected after the reproduction of the at least one voice message.

In one embodiment, the wearable electronic device is further configured to measure a time elapsed since the reproduction of the at least one voice message.

When a predetermined time has elapsed without having detected the touch of the user, the wearable electronic device reproduces the at least one voice message again, increments a reproduction counter by the at least one voice message, if the reproduction counter reaches a predetermined number, it does not further reproduce the at least one voice message, and communicates to the software application that the predetermined number has been reached.

The software application is configured to identify a vexing event experienced by the user when information is received that the predetermined number has been reached.

The above-mentioned embodiments make it possible to determine with greater precision and detail the situations in which the user experiences troubles in carrying out a scheduled activity.

In one embodiment, the wearable electronic device is configured to count a number of events in which:
a. the time elapsed between the reproduction of the at least one voice message and the touch detection exceeds a minimum value, and
b. the consecutive reproductions of the message exceed a minimum number before the touch is detected.

The wearable electronic device communicates the number of events to the software application, which is configured to signal the at least one voice message as ineffective.

Thanks to the analysis of the reactions of the user to the voice messages, it is possible to easily and clearly identify which voice messages are not effective in promoting the performance of the scheduled activity. This allows the caregiver to verify whether the autistic user is not interested in the execution of the activity and whether the problem lies in the lack of precise indications or in the lexical choice in the voice message. Consequently, it is possible to limit, if not eliminate altogether, the frustration in both the user and in the caregiver connected to the trouble of carrying out the envisaged activity by effectively editing the voice message.

In one embodiment, the system further comprises detection means configured to measure at least one physiological parameter of the user and to transmit the measurement of the at least one physiological parameter to the software application.

In this case, the software application is configured to identify a vexing event experienced by the user in a time interval comprising said time instant based on the measurement of the at least one physiological parameter provided by the detection means.

The use of the user's physiological information makes it possible to identify with precision the most pronounced vexing events, particularly meltdown episodes in the case of autistic users. The caregiver is thus able to intervene promptly when actually needed by the user, identify activities that cause excessive stress and provide targeted care measures and/or optimise the scheduling of activities so as to reduce the stress experienced by the user. In addition, the system allows to reduce the time of direct care by the guardian and/or by the healthcare professional, thus reducing a cost in terms of time and/or economic resources needed to adequately assist the user.

In one embodiment, the software application is configured to generate an alert message when a vexing event is identified.

This allows the guardian and/or the healthcare professional to learn in real time when the user has a real need for direct assistance, particularly in the case of more severe vexing events.

In one embodiment, the software application is configured to:
- record the at least one voice message comprising an invitation to carry out an activity, and
- record the at least one further voice message comprising an instruction to be followed to carry out said activity.

Preferably, the software application is configured to present an interface through which an operator can select the time instant associated with said voice message and start recording the at least one voice message.

In this way, highly personalised messages can be prepared that allow to best guide the user to carry out the corresponding activity.

In one embodiment, the agenda comprises a time interval in which the activity should be performed. In such a case, the wearable electronic device is configured to signal to the user at least once, a time remaining to complete the activity within said time interval.

In one embodiment, the detection means comprise a sensor configured to measure the electrical conductivity of the skin of the user as a function of time. In this case, the software application is configured to identify a vexing event when the conductivity measurement exceeds a threshold value.

The Applicant has noted that the measurement of the electrical conductivity of the skin allows the effective and reliable identification of vexing events to which the user is subjected, in particular meltdown events of autistic users.

In one embodiment, the detection means comprise at least one further sensor configured to measure one of:
- a movement of the user,
- a blood volume pulse of the user, and
- a frequency and/or a variability of the heartbeat of the user.

In this case, the software application is configured to identify a vexing event when it is detected at least one among:
- a repetitive movement performed by the user,
- a blood volume pulse above a threshold value,
- a deviation of a trend of the blood volume pulse from an expected trend, and
- a frequency and/or a variability of the heartbeat above the respective threshold values,
in addition to exceeding the threshold value by the measurement of the electrical conductivity of the skin of the user.

The Applicant has determined that combining the measurements relative to the electrical conductivity of the skin with the measurements listed above makes it possible to identify even more effectively and with greater reliability vexing events to which the user is subject, in particular meltdown events of autistic users. In addition, the greater amount of information thus obtained makes it possible to obtain a better understanding of the vexing event and thus to study better strategies to avoid or at least mitigate the vexing event.

In an alternative embodiment, the detection means comprise at least one further sensor configured to measure an ambient temperature surrounding the user, i.e. in proximity of the user.

In this case, the software application is configured to modify at least one of:
- the measurement of the electrical conductivity of the skin of the user, and
- the threshold value associated with the electrical measurement of the electrical conductivity of the skin of the user,
when an ambient temperature above a threshold ambient temperature value or an ambient temperature outside an interval of expected ambient temperature values is measured.

Additionally or alternatively, the detection means comprise at least one further sensor configured to measure a body temperature surrounding the user.

In this case, the software application is configured to modify at least one of:
- the measurement of the electrical conductivity of the skin of the user, and
- the threshold value associated with the electrical measurement,
when a body temperature above a threshold body temperature value or an ambient temperature outside an interval of expected body temperature values is measured.

Optionally, one or more other measurements or relative threshold values may also be altered based on the measured ambient temperature and/or body temperature such as, for example, the heart rate measurement or the threshold value associated therewith.

This makes it possible to detect ambient and/or body conditions (such as fever) that can alter one or more measurements related to the user, in particular the measurement of the electrical conductivity of the skin. This thus makes it possible to more reliably assess the occurrence of a meltdown event by avoiding false positives due to measurements that are outside the threshold because of an excessive ambient and/or body temperature or outside an interval of values that is considered normal.

In one embodiment, at least a portion of the detection means are integrated in the wearable electronic device.

Alternatively, the detection means are included in a further wearable device worn by the user.

Preferably, the wearable electronic device is a necklace, while the further wearable electronic device is a bracelet.

The Applicant has noted that the use of a necklace and of the bracelet was easily acceptable to the user. Furthermore, the voice message transmitted by the necklace is emitted from a source in a position similar to the mouth position of a user's interlocutor, thereby allowing a more natural experience by the user listening to the voice message.

As a further alternative or addition, at least part of the detection means are integrated in an object used by the user during a vexing event, in at least one tool used by the user during the execution of said activity to be carried out, and/or comprise sensors suitable for detecting the presence of the user or of the wearable electronic device positioned in a place where said activity to be carried out is to be carried out.

The use of detection means located in objects or in places usually used by the user makes it possible to acquire more complete information on the status of the user during the performance of the activity and/or in the event of a vexing event.

A different aspect of the present invention concerns a method for assisting the execution of programmed activities comprising the steps of:
- programming an activity agenda through a software application, the activity agenda comprising at least one voice message and a time instant associated with said voice message, said at least one voice message comprising an invitation to carry out an activity,
- transmitting the at least one voice message and the time instant associated with the wearable electronic device worn by a user,
- reproducing, by means of the wearable electronic device, the at least one voice message at the time instant associated with the at least one voice message,
- detecting an at least one touch of an interactive portion of the wearable electronic device subsequent to the reproduction of the at least one voice message,
- measuring a duration of the at least one touch,
- transmitting a number of detected touches and the measured duration of each detected touch to the software application, and
- identifying a vexing event experienced by the user when it is detected at least one between:
   a touch lasting longer than a threshold duration value, and
   a sequence of consecutive touches within a predetermined time interval.

In one embodiment, the method further comprises the steps of:
- measuring at least one physiological parameter of the user by means of detection means, and
- identifying a vexing event in a time interval comprising the time instant associated with the at least one voice message based on said measurement of the at least one physiological parameter.

The method makes it possible to obtain advantages similar to those set out above in relation to the system described above.

Further features and advantages of the present invention will be more evident from the description of the accompanying drawings.

### BRIEF DESCRIPTION OF THE DRAWINGS

The invention is described hereinbelow with reference to certain examples provided by way of non-limiting example and illustrated in the accompanying drawings. These drawings illustrate different aspects and embodiments of the present invention and reference numerals illustrating structures, components, materials and/or similar elements in different drawings are indicated by similar reference numerals, where appropriate.
Figure 1 illustrates an assistance system for an autistic user according to an embodiment of the present invention;
Figure 2A is an axonometric view of a pendant included in the system of Figure 1;
Figure 2B is a block diagram of the electronics integrated in the pendant of Figure 2A;
Figure 3A is an axonometric view of a bracelet included in the system of Figure 1;
Figure 3B is a block diagram of the electronics integrated in the bracelet of Figure 3A;
Figure 4 is an axonometric view of the pendant and of the bracelet coupled to a charging device;
Figures 5A-5D schematically illustrate screens generated by a software application included in the system of Figure 1;
Figure 6 is a flow chart of a procedure for stimulating a user to perform an activity according to an embodiment of the present invention;
Figure 7 is a flow chart of a procedure for stimulating a user to perform an activity according to a different embodiment of the present invention, and
Figure 8 is a flow chart of a procedure for monitoring the user's physiological parameters.

### DETAILED DESCRIPTION OF THE INVENTION

While the invention is susceptible to various modifications and alternative constructions, certain preferred embodiments are shown in the drawings and are described hereinbelow in detail. It must in any case be understood that there is no intention to limit the invention to the specific embodiment illustrated, but, on the contrary, the invention intends covering all the modifications, alternative and equivalent constructions that fall within the scope of the invention as defined in the claims.

The use of "for example", "etc.", "or" indicates non-exclusive alternatives without limitation, unless otherwise indicated. The use of "includes" means "includes, but not limited to" unless otherwise indicated.

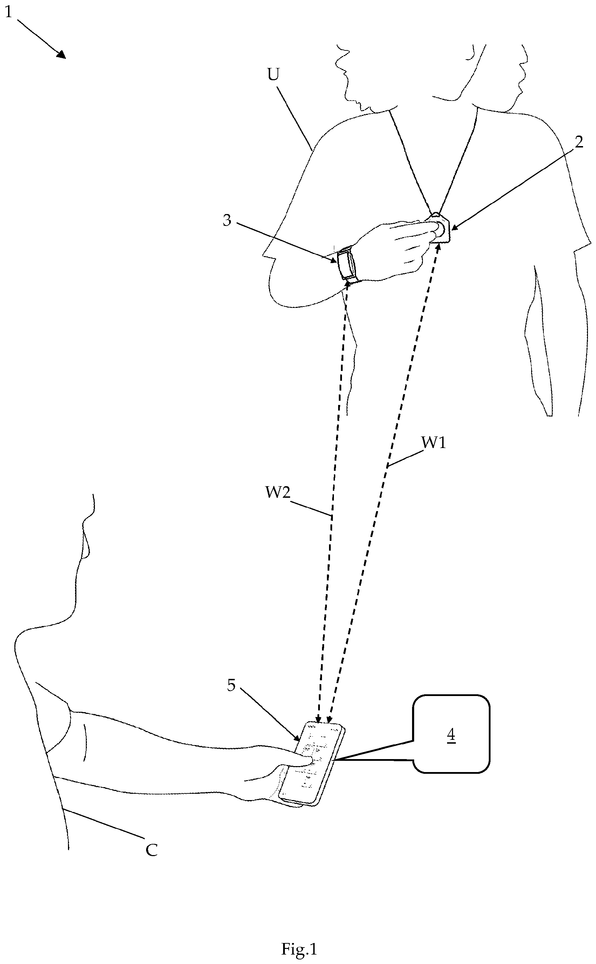

With reference to Figure 1, an assistance system for autistic users is described according to an embodiment of the present invention, referred to simply as "system 1" below.

The system 1 comprises a first user device, a necklace 2 in the illustrated example, and a second user device, a bracelet 3 in the illustrated example. In use, the necklace 2 and the bracelet 3 are worn by a user U suffering from an autism spectrum disorder. Furthermore, the system 1 comprises a software application, hereinafter referred to as "app 4", installed on a remote terminal, a smartphone 5 in the illustrated example, of an assistant, or caregiver C, of the user U.

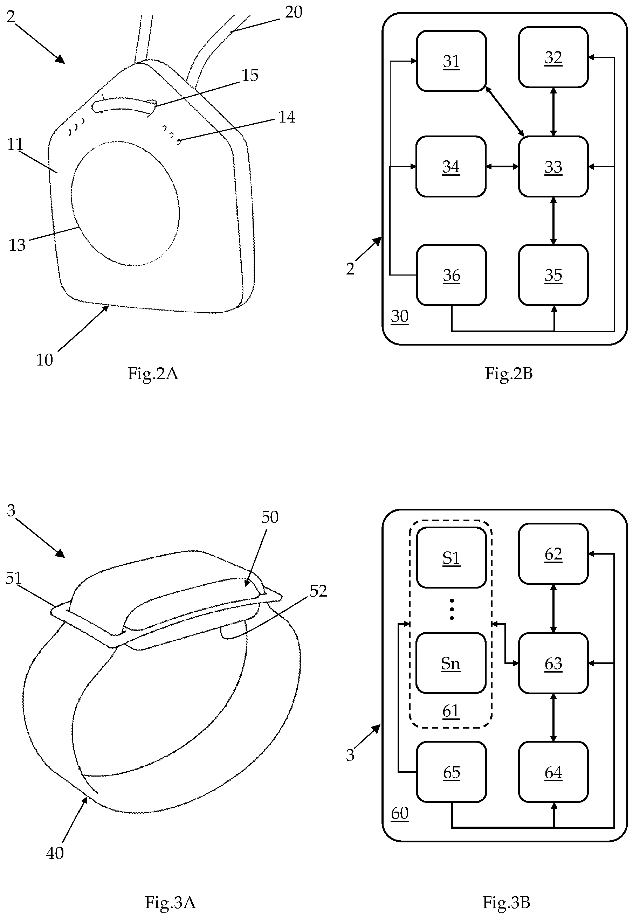

With particular reference to Figures 2A and 2B, the necklace 2 comprises a pendant 10 and a ribbon 20. In particular, the pendant 10 comprises a casing 11, preferably waterproof, which houses an electronic circuit 30 (illustrated schematically in Figure 2B). For example, the casing 11 is formed by a pair of shells constrained to each other by means of mechanical and/or magnetic mutual fasteners (not visible in the figures). Preferably, the casing 11 has a rounded shape or in any case has no sharp vertices and edges.

Furthermore, the casing 11 comprises an interactive portion 13 coupled to a touch sensor 31, preferably of a capacitive type, housed inside the casing 11. In a preferred embodiment, the interactive portion 13 is made with a texture and/or a colour that is easily distinguishable from the remaining part of the casing 11. For example, the interactive portion 13 is formed of wood and, optionally, is coloured in soft shades. Preferably, the interactive portion has a discoidal shape.

Preferably, the casing 11 comprises holes 14 and/or thinned portions formed at a loudspeaker of a sound synthesis module 32 included in the electronic circuit 30, in order to allow optimal propagation of acoustic vibrations produced by the loudspeaker of the sound synthesis module 32.

Finally, the casing 11 comprises a pair of through holes 15 that allow the insertion of the ribbon 20, which is used to wear the pendant 2 around the neck of the user U.

In addition to the touch sensor 31 and the sound synthesis module 32, the electronic circuit 30 of the pendant 2 comprises a control module 33 - preferably, comprising a microcontroller -, a memory module 34, a communication module 35 - capable of exchanging data with the app 4 via a wireless communication channel W1 - and a power supply module 36 - comprising a battery. In particular, the control module 33 is connected to at least the modules 31, 32, 34 and 35 and is configured to govern the operation thereof. In addition, the power supply module 36 is connected to the modules 31, 32, 33, 34 and 35 and is configured to deliver operating electric power thereto.

The bracelet 3 comprises a band 40, preferably with a magnetic clasp (not visible), and a casing 50, preferably waterproof, which encloses an electronic circuit 60. For example, the casing 50 is formed by a pair of shells constrained to each other by means of mechanical and/or magnetic mutual fasteners (not visible in the figures).

The casing 50 comprises a fastener 51 to enable a coupling between the band 40 and the casing 50. In the example illustrated, the fastener 51 is a protruding frame comprising a pair of through holes suitable for receiving the band 40.

Finally, the casing 50 comprises a surface 52 configured to contact the epidermis of the user U when the bracelet 3 is worn. Advantageously, the surface 52 comprises one or more holes and/or windows through which one or more sensors S1-Sn of a sensor assembly 61 included in the electronic circuit can make measurements of physiological parameters of the user U.

In particular, the sensor assembly 61 comprises at least one galvanic skin response sensor (or GSR sensor). Preferably, the sensor assembly also comprises at least one of:
- a photoplethysmography (or PPG sensor);
- an accelerometer;
- a magnetometer;
- an ambient temperature sensor, and
- a body temperature sensor.

In addition to the sensor assembly 61, the electronic circuit 60 of the bracelet 3 comprises a control module 62 - preferably, comprising a microcontroller -, a memory module 63, a communication module 64 - capable of transmitting data to the app 4 via a wireless communication channel W2 - and a power supply module 65 - comprising a battery. In particular, the control module 62 is connected to at least the modules 61, 63, 64 and is configured to govern the operation thereof. In addition, the power supply module 65 is connected to the modules 61, 62, 63 and 64 and is configured to deliver operating electrical power thereto.

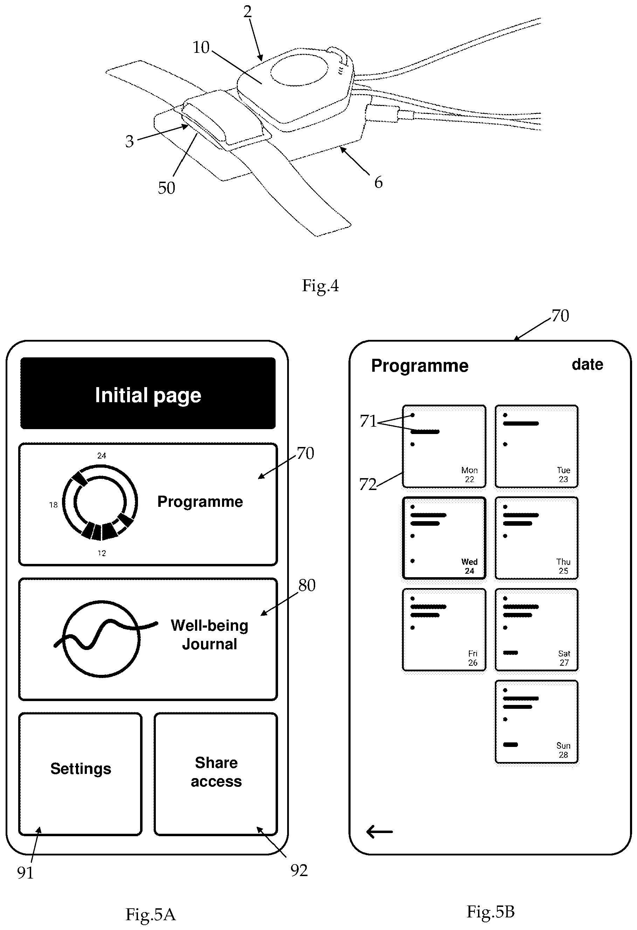

Optionally, the system 1 comprises a wireless charging device 6 (illustrated in Figure 4). Advantageously, the wireless charging device 6 comprises a shaped housing for receiving the casing 10 of the pendant 2 and a shaped housing for receiving the casing 50 of the bracelet 3.

The app 4 is configured to perform an activity programming function, i.e. an activity agenda 70 through which the caregiver C can set a set of activities - e.g. getting dressed, eating a meal, personal hygiene activities - that the user U should perform starting from respective initial time instants and, preferably, conclude within a predetermined time period.

For example, through a graphical interface of the app 4 - some screens of which are illustrated in Figures 5A-5D - the caregiver C can initiate the activity agenda 70 from a home screen, illustrated in Figure 5A. Through the app interface - illustrated in Figure 5B - the caregiver C is able to define one or more activities 71 to be performed starting from an initial time instant *t₀* - for example, a predetermined time of a selected day 72. Advantageously, the activity agenda 70 is configured to allow the caregiver C to record a respective voice message to be associated with each of the programmed activities 71 and/or to associate a stored previous voice message with each of the programmed activities 71.

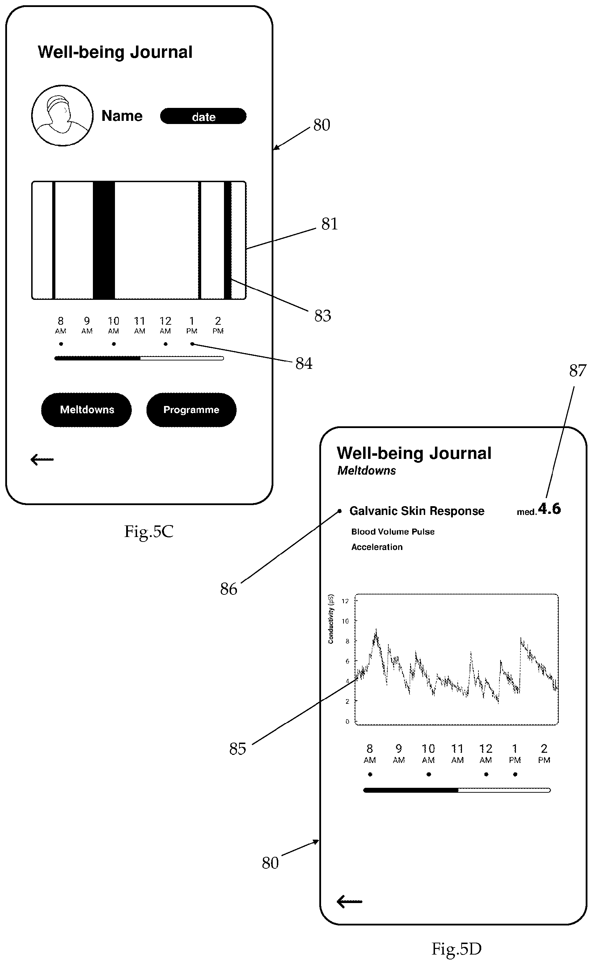

The app 4 is also configured to receive, analyse and process the measurements taken by the bracelet 3. Preferably, the app 4 is configured to generate a log 80 (or journal) of information on the user U accessible through the graphical interface - Figures 5A, 5C and 5D.

In the preferred embodiment, the app 4 is configured to display the information contained in the log 80 in graphical form. As illustrated in the example of Figure 5C, the log 80 generates a graph 81 in which vexing and/or stress events are indicated - for example, in the case of autistic users, particularly intense vexing events are indicated by the term 'meltdown' 83 - as a function of time. Preferably, the envisaged activities 84 are also indicated as a function of time.

In addition, the app 4 allows the display of detailed information relative to each vexing event 83. As illustrated in the example of Figure 5C, the app 4 is configured to display a graph 85 as a function of time of at least one selected physiological parameter 86. Preferably, the app 4 is configured to display one or more additional information related to the selected physiological parameter 86. For example, the app 4 is configured to calculate and display an average value 87 of the selected physiological parameter 86 over a predetermined time period, such as the duration of a vexing event 83.

Optionally - as visible in Figure 5A -, the app 4 comprises one or more additional functions, for example a setting adjustment function 91 and a sharing function 92 that allows information about the user U to be shared with a third party.

The system 1 allows to perform a method of assistance to the user U in order to achieve a substantially autonomous management of the daily activities to be carried out.

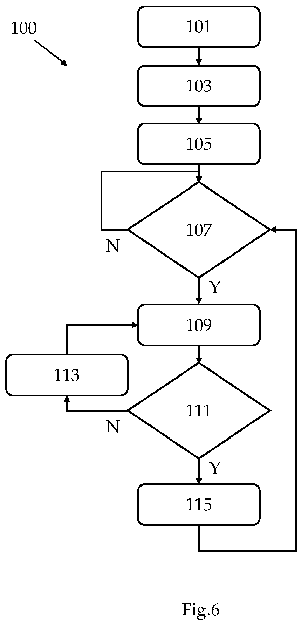

Specifically, the method comprises a procedure 100 - of which Figure 6 is a flow chart - of stimulation involving the following steps.

Initially, the caregiver C defines by means of the activity agenda 70 of the app 4 a set of activities 71 that should be performed by the user U starting from corresponding initial time instants *t₀* and, preferably, concluded within a corresponding predetermined time period *T_{A}* (block 101).

For each defined activity 71, the caregiver C records a corresponding voice message or selects a previous stored voice message (block 103). Advantageously, the voice message contains an instruction, or prompt, prompting the user U to undertake the associated activity 71. For example, if the envisaged activity is an oral hygiene operation, the voice message may be of the type: "[username U], it's time to brush your teeth; go to the bathroom and brush your teeth.".

The programmed activities 71 and the related recorded voice messages are transmitted from the smartphone 5 of the caregiver C to the necklace 2 (block 105).

The electronic circuit 30 of the necklace 2 is configured to verify whether a current time instant tcorresponds to the initial time instant to associated with one of the programmed activities 71 (decision block 107 and output branch N of block 107).

In the event that the current time instant is within the activity time period *T_{A}* of one of the programmed activities 71 (output branch Y of block 107), the electronic circuit 30 of the necklace 2 is configured to emit an acoustic signal (block 109) to draw the attention of the user U.

Next, the electronic circuit 30 of the necklace 2 is configured to detect an interaction of the user U with the interactive portion 13 of the pendant 10 (decision block 111), preferably within a waiting time interval *ΔT.* In other words, it is verified whether the user U touches the interactive portion 13 of the pendant 10.

If a touch of the user U is not detected within the waiting time interval *ΔT* (output branch N of block 111), the acoustic signal is emitted again (returning to block 109) after a predetermined delay time r has elapsed - for example of the order of minutes - (block 113).

Conversely, if the touch of the user U is detected within the waiting time interval *ΔT* (output branch Y of block 111), the electronic circuit 30 of the necklace 2 is configured to reproduce the voice message associated with the programmed activity 71 to be performed in the activity time period *T_{A}* starting from the initial time instant *t₀* (block 115).

Subsequently, the procedure 100 is reiterated to identify other initial time instants *t₀* associated with each other programmed activity 71 - that is, the procedure 100 is reiterated starting from the step described in relation to block 107.

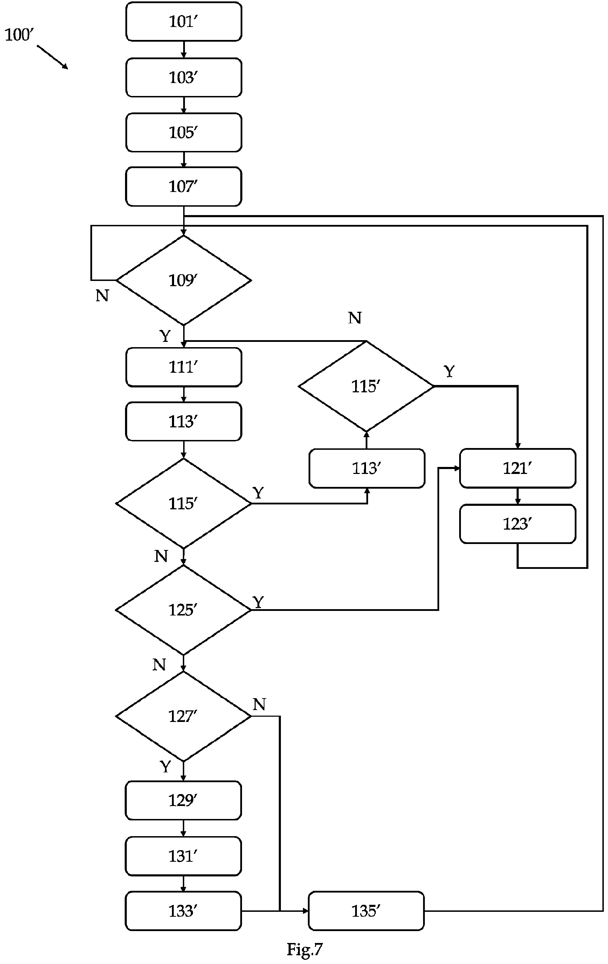

In an alternative embodiment, the method comprises an alternative procedure 100' of stimulation that involves the following steps - of which Figure 7 is a flow chart.

Initially, the caregiver C defines the one or more activities 71 that should be carried out by the user U starting from corresponding initial time instants *t₀* and, preferably, concluded within a corresponding predetermined time period *T_{A}* by means of the activity agenda 70 of the app 4 (block 101').

For each of these activities 71, the caregiver C records a corresponding initial voice message or selects a stored previous initial voice message (block 103'). Advantageously, the initial voice message contains an indication, or pre-prompt, of the activity to be undertaken by the user U. For example, if the envisaged activity is an oral hygiene operation, the voice message may be of the type: "[username U], it's time to brush your teeth".

In addition, the caregiver C records one or more action voice messages or selects one or more stored previous action voice messages (block 105'). Each action voice message contains an instruction, or prompt, describing an action to be performed in order to complete the activity - in the example considered above, a sequence of action voice messages may be of the type: "Go to the bathroom", "Apply toothpaste on your toothbrush" "Massage your teeth with your toothbrush", etc. In other words, the activity to be performed is divided into individual actions to be carried out in sequence and a corresponding action voice message is recorded for each action.

The programmed activities 71 and the related recorded voice messages are transmitted from the smartphone 5 of the caregiver C to the necklace 2 (block 107').

The electronic circuit 30 of the necklace 2 is configured to verify whether a current time instant tcorresponds to the initial time instant *t₀* associated with one of the programmed activities 71 (decision block 109' and output branch N of block 109').

When the initial time *t₀* (output branch Y of block 109') is reached, the electronic circuit 30 of the necklace 2 is configured to emit the initial voice message (block 111') to draw the attention of the user U and inform him about the activity to be carried out.

In addition, the electronic circuit 30 of the necklace 2 is configured to measure an elapsed time *Δ*t since the emission of the initial voice message (block 113'). The electronic circuit 30 of the necklace 2 is configured to detect an interaction of the user U with the interactive portion 13 of the pendant 10 (decision block 115'), preferably within a waiting time interval *ΔT.* In other words, it is verified whether the user U touches the interactive portion 13 of the pendant 10.

If a touch of the user U is not detected within the waiting time interval *ΔT* (output branch N of block 115'), a message repetition counter is incremented (block 117') and it is verified whether the message repetition counter has exceeded a limit value (decision block 119').

If the message repetition counter has not exceeded the limit value (output branch N of block 119'), the initial voice message is emitted again (returning to block 111').

Otherwise, if the message repetition counter exceeds the limit value (output branch Y of block 119'), the electronic circuit 30 of the necklace 2 is configured to interrupt the reproduction of the voice messages connected to the current activity (block 121') and transmits to the app 4 on the smartphone 5 an indication of a vexing event by the user in carrying out the activity (block 123'). Preferably, in this case the indication of a vexing event comprises information related to the fact that the initial voice message has been repeated a maximum number of times without feedback from the user.

Subsequently, the electronic circuit 30 of the necklace 2 is configured to verify whether an initial time instant *t₀* associated with a different activity has been reached (returning to block 109').

Returning to the decision block 115', if a touch of the user U is detected within the waiting time interval *ΔT* (output branch Y of the block 115'), the electronic circuit 30 of the necklace 2 is configured to verify whether the touch of the user had a duration equal to or greater than a limit duration *T_{L}* and/or whether a sequence of touches comprising a number of touches equal to or greater than a limit number *n_{L}*, possibly, within a predetermined time interval (decision block 125') has been detected. In the affirmative case (output branch Y of block 125'), the electronic circuit 30 of the necklace 2 is configured to interrupt the reproduction of the voice messages connected to the current activity and transmits to the app 4 on the smartphone 5 an indication of a vexing event on the part of the user in carrying out the activity (that is, the procedure passes to blocks 121' and 123'). Preferably, in this case the vexing event indication comprises information related to the fact that the activity has been interrupted due to an abnormal reaction of the user.

If, on the other hand, a number of touches less than the limit number *n_{L}* and/or a duration less than the limit duration (output branch N of block 125') has been detected, the electronic circuit 30 of the necklace 2 is configured to verify whether the elapsed time *Δt* since the emission of the initial voice message is equal to or greater than a minimum time interval *Tₘ* and/or whether the message repetition counter is equal to or greater than a minimum number of repetitions *rₘ* (decision block 127'). In the affirmative case (output branch Y of block 127'), the electronic circuit 30 of the necklace 2 is configured to transmit to the app 4 on the smartphone 5 an indication of a minor vexing event on the part of the user (block 131') and the app 4 is configured to signal that the at least one voice message associated with the current activity is ineffective or can be improved (block 133').

If the elapsed time *Δt* is less than the minimum time interval *Tₘ* and/or if the message repetition counter is less than a minimum number of repetitions *rₘ* (output branch N of block 127') or after execution of step at block 133', the electronic circuit 30 of the necklace 2 is configured to reproduce the action voice messages according to the sequence and timings defined by the caregiver C (block 135'). Thereafter, i.e. once the current activity has been completed, the procedure 100' is reiterated from block 109' to verify whether an initial time instant *t₀* associated with a different activity has been reached.

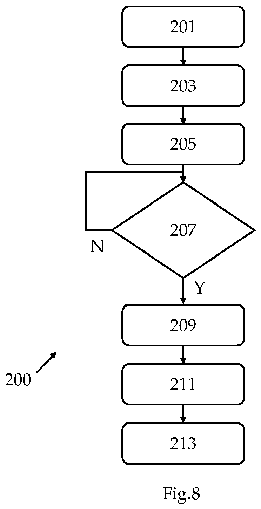

In parallel to the procedure 100 or 100' the method provides for performing a procedure 200 for monitoring the physiological parameters of the user U - of which Figure 8 is a flow chart.

The procedure 200 provided for the electronic circuit 60 of the bracelet 3 to continuously or periodically measure at least one physiological parameter of the user (block 201).

In the preferred embodiment, the electrical conductivity of the skin of the user U is measured. Optionally, at least one of the following is measured:
- the movement of the user U;
- the blood volume pulse (BVP);
- the body temperature of the user U, and
- the heartbeat of the user U.

The electronic circuit 60 of the bracelet 3 is configured to transmit to the smartphone of the caregiver C, preferably periodically, one or more measurements made for each physiological parameter detected (block 203).

Finally, the app 4 is configured to store the measurements received from the bracelet 3 (block 205).

Subsequently, the measurements received are processed to obtain information on the user U. In particular, the procedure 200 involves identifying the occurrence of a greater vexing event, such as a meltdown 83 of the user U (decision block 207 and output branch N thereof).

In particular, the app 4 is configured to identify a probable meltdown event 83 when the measurement of the electrical conductivity of the skin of the user U exceeds a limit value - for example, exceeds a threshold conductivity value - for a time period greater than or equal to a stress time interval Δt_{S}.

In the preferred embodiment, the app 4 is configured to identify a meltdown event 83 when, in addition to the change in the electrical conductivity, at least one of the following is identified:
- a repetitive movement performed by the user U (or motor stereotypy) for a time period greater than or equal to a stress time interval;
- a change in ambient temperature above a threshold value, e.g. such that sweating and/or the heartbeat of the user increase, affecting the electrical conductivity and heart rate measurements. ;
- a body temperature and/or a change in the body temperature of the user above a threshold value;
- a blood volume pulse trend deviating from an expected trend or exceeding a threshold value, and
- a frequency and/or a variability of the heartbeat.

When a meltdown event 83 (output branch Y of block 207) is identified, it is preferably calculated and, preferably stored, (block 209) at least one of:
- an indication of the intensity of the meltdown event 83 calculated on the basis of one or more of the measurements taken through the bracelet 3;
- a duration of the meltdown event 83, and
- one or more maximum, minimum and/or average values of the measurements taken during the meltdown event 83.

In addition, the app 4 is configured to determine and, preferably store, whether the meltdown event 83 occurred at the neighbourhood of the initial time instant *t₀* associated with a programmed activity 71. Herein, by neighbourhood of the initial time instant *t₀* it is meant a time interval comprising the initial time instant *t₀* of the start and, possibly, the entire time period of activity *T_{A}* associated with the corresponding programmed activity 71 (block 211).

In series or in parallel with the steps described in relation to blocks 209 and 211, the app 4 is configured to generate an alert message (block 213) whose aim is to alert the caregiver C of the occurrence of the identified meltdown event 83.

For example, the alert message comprises the reproduction of light and/or acoustic signals and, preferably, the display of one or more information about the meltdown event 83 identified through the user interface of the smartphone 5. Examples of information on the meltdown event 83 comprise, but are not limited to, indicating the intensity of the meltdown event, a start time instant of the meltdown event and/or one or more values measured by the bracelet 3.

However, it is clear that the above examples must not be interpreted in a limiting sense and the invention thus conceived is susceptible of numerous modifications and variations.

For example, although a pair of user devices, namely the necklace and the bracelet, have been described, there is nothing to prevent a single wearable electronic device that integrates the functionality of both electronic devices, e.g. a bracelet or a chest strap from being created.

In an alternative embodiment (not illustrated), the necklace pendant also comprises a light source - for example, one or more LEDs - configured to emit a visual signal in addition to, or alternatively to, the acoustic signal warning the user of the presence of a voice message to be heard.

In an alternative embodiment (not illustrated), the system comprises a remote unit, for example a server, configured to receive, store and/or process information provided by the user devices and/or by the terminal of the caregiver.

As will be apparent to the person skilled in the art, one or more of the steps of the procedures 100, 100' and 200 described above may be performed in parallel with each other - such as the steps relative to blocks 209, 211 and 213 of the procedure 200 - or in a different order from the one presented above. Similarly, one or more optional steps can be added or removed from one or more of the procedures described above. For example, one or two of steps 117' and 119', or step 125', or steps 127' - 133'.

In one embodiment (not illustrated), the stimulation procedure provides for signalling, for example by means of a specific pre-recorded message, at least once, a time remaining to complete the programmed activity during the corresponding period of execution of that activity.

Additionally or alternatively, the stimulation procedure may provide for the user to confirm the completion of the programmed activity, for example, by interacting with the interactive portion of the pendant.

In one embodiment (not illustrated), an alternative stimulation procedure involves verifying the execution of each of the suggested actions by means of an action voice message by touching the interactive portion of the pendant before moving on to the reproduction of the next action voice message. Preferably, the alternative stimulation procedure involves identifying vexing events based on the user's interaction with the interactive portion of the pendant in a manner similar to that described above in relation to one or more of steps 117' and 119', step 125', steps 127' - 133' of the procedure 100'.

In an alternative embodiment (not illustrated), the procedure for monitoring the user's physiological parameters comprises the alert message to be generated only if the meltdown event is associated with measurements of one or more physiological parameters above respective alert values - greater than or equal to the threshold values identifying a meltdown event. In addition or alternatively, the alert message can only be emitted if the measurements of one or more physiological parameters are greater than the threshold values or, alternatively, the alert values for a time period greater than or equal to a predetermined time period.

Additionally or alternatively, the monitoring procedure is configured to apply a correction value to the measurements of the electrical conductivity of the skin and/or of the heart rate when an ambient temperature above the threshold temperature value - or outside an interval of permitted temperatures - such as to alter the electrical conductivity of the skin and/or of the heart rate is detected. As a further addition or alternative, the detection of a body temperature above a respective threshold temperature value - or outside a permitted body temperature interval - can also lead to the application of a respective correction value to the measurements of the electrical conductivity of the skin and/or of the heart rate.

Alternatively, instead of making a correction to the electrical conductivity and/or heart rate measurements, the monitoring procedure may provide for applying a correction to the threshold values of such measurements considered indicative of a meltdown event.

The procedures 100, 100' and 200 presented above form a method for assisting an autistic user. In addition, one or more steps of the same procedure or of different procedures may be performed in parallel between each other or according to a different order from the one presented above. Similarly, one or more optional steps may be added or removed from one or more of the above-described procedures.

Naturally, all the details can be replaced with other technically equivalent elements.

In particular, the necklace and the bracelet may be configured to establish a direct, preferably short-range, communication with the terminal of the caregiver, for example via a Bluetooth^{®} communication channel. In addition or alternatively, the necklace and the bracelet can be configured to establish an indirect communication with the terminal of the caregiver, e.g. via a WiFi communication channel or based on a mobile telephone network.

Again, nothing prevents a first user device between the necklace and the bracelet from being configured to establish a communication channel with the other user device - i.e., the bracelet and the necklace, respectively - for exchanging data. In this case, it is possible that only one between the necklace and the bracelet is configured to establish a communication channel with the remote terminal of the caregiver and that it is configured to exchange data generated and/or directed to both user devices.

It will also be apparent that the remote terminal of the caregiver may comprise a device other than a smartphone, such as a tablet, a smartwatch, a personal computer, etc.

As a further alternative or addition, the system may comprise an object used by the user during a vexing event, such as an anti-stress ball, or one or more utensils used by the user during the performance of activities - for example, kitchen utensils. These objects are provided with sensors to detect the interaction by the user. In addition, the system may comprise sensors suitable for detecting the presence of the user or of the pendant positioned in a place where the activities are to be carried out.

Again, even where not expressly stated, it will be clear that the detection of exceeding a threshold value can be replaced by detecting a measurement going outside an interval of expected/admissible values, or by detecting a measurement below a threshold value.

Similarly, instead of considering a single measurement, it is possible to consider more than one measurement, e.g. a predetermined number of measurements that are consecutive or within a time interval of a predetermined duration, above the threshold value (or outside the expected/admissible values) provided by the same sensor to identify a meltdown condition.

Furthermore, although the example of an autistic user has been given in the above description, it will be evident that the proposed system can be adapted to provide assistance to users suffering from different pathologies and/or disorders whose evolution/trend can be monitored through the analysis of the above-mentioned measurements and/or other measurable physiological parameters. For example, the system can be configured to assist users with AD-HD (Attention Deficit and Hyperactive Disorder) in carrying out daily activities (e.g.: body care) or work activities (e.g.: preparing a dish following a recipe).

In conclusion, the materials used, as well as the contingent shapes and dimensions of the aforementioned devices, apparatuses and terminals, may be any according to the specific implementation requirements defined by the scope of protection of the following claims.

## Claims

1. System (1) for assisting the execution of programmed activities, the system (1) comprising:
- a wearable electronic device (2), wearable by a user, comprising a sound reproduction module (32) and an interactive portion (13, 31) configured to detect a touch of the user, and
- a software application (4) running on a remote terminal (5) suitable for exchanging data with the wearable electronic device (2),
wherein the software application (4) comprises an agenda (70) that stores at least one voice message, said voice message comprises an invitation to carry out an activity and is associated with a time instant, the software application (4) being configured to transmit the at least one voice message and the time instant associated therewith to the wearable electronic device (2), and
wherein
the wearable electronic device (2) is configured to reproduce the at least one voice message at the time instant associated with the at least one voice message,
**characterised in that**
the wearable electronic device (2) is further configured to
- detect at least one touch of the interactive portion (13, 31) following the reproduction of the at least one voice message,
- measure a duration of the at least one touch,
- transmit to the software application (4) a number of detected touches and the measured duration of each detected touch, and
**in that**
the software application (4) is configured to identify a vexing event experienced by the user when it is detected at least one between:
a touch lasting longer than a threshold duration value, and
a sequence of consecutive touches within a predetermined time interval.

2. System (1) according to claim 1, wherein the agenda (70) stores at least one further voice message containing an instruction to be followed to complete said activity, the software application (4) being configured to transmit said at least one further voice message to the wearable electronic device, and
wherein the wearable electronic device (3) is configured to reproduce said at least one further voice message when a touch of duration equal to or shorter than said threshold duration is detected after the reproduction of the at least one voice message.

3. System (1) according to claim 2, wherein the wearable electronic device (2) is configured not to reproduce said at least one further voice message when a touch lasting longer than said threshold duration is detected after the reproduction of the at least one voice message.

4. System (1) according to any one of the preceding claims, wherein the wearable electronic device (2) is further configured to:
- measure a time elapsed since the reproduction of the at least one voice message,
when a predetermined time has elapsed without having detected the touch of the user, the wearable electronic device (2):
- reproduces the at least one voice message again,
- increments a reproduction counter by the at least one voice message,
- if the reproduction counter reaches a predetermined number, it does not further reproduce the at least one voice message, and
- communicates to the software application (4) that the predetermined number has been reached, and wherein the software application (4) is configured to identify a vexing event experienced by the user when information is received that the predetermined number has been reached.

5. System (1) according to claim 4, wherein the wearable electronic device (2) is configured to:
- count a number of events in which:
a. the time elapsed between the reproduction of the at least one voice message and the touch detection exceeds a minimum value, and
b. the consecutive reproductions of the message exceed a minimum number before the touch is detected, and
- communicate said number of events to the software application (4), and
wherein the software application (4) is configured to signal the at least one voice message as ineffective.

6. System (1) according to any one of the preceding claims, wherein the system further comprises detection means (60) configured to measure at least one physiological parameter of the user and to transmit the measurement of the at least one physiological parameter to the software application (4), and
wherein the software application (4) is configured to identify a vexing event experienced by the user in a time interval comprising said time instant based on the measurement of the at least one physiological parameter provided by the detection means (60).

7. System (1) according to any one of the preceding claims, wherein the software application (4) is configured to generate an alert message when the vexing event is identified.

8. System (1) according to claim 2, wherein the software application (4) is configured to:
- record the at least one voice message comprising an invitation to carry out an activity, and
- record the at least one further voice message comprising an instruction to be followed to carry out said activity.

9. System according to claim 8, wherein the software application (4) is configured to:
- present an interface (70) through which an operator can select the time instant associated with the voice message and start recording the at least one voice message.

10. System (1) according to any one of the preceding claims, wherein the detection means (60) comprise a sensor (61) configured to measure the electrical conductivity of the skin of the user as a function of time, and
wherein the software application (4) is configured to identify a vexing event when the conductivity measurement exceeds a threshold value.

11. System (1) according to claim 10, wherein the detection means comprise at least one further sensor (61) configured to measure one of:
- a movement of the user,
- a blood volume pulse of the user, and
- a frequency and/or a variability of the heartbeat of the user, and
wherein the software application (4) is configured to identify a vexing event when it is detected at least one among:
- a repetitive movement performed by the user,
- an increase in ambient temperature above a threshold ambient temperature,
- a blood volume pulse above a threshold value,
- a deviation of a trend of the blood volume pulse from an expected trend, and
- a frequency and/or a variability of the heartbeat,
in addition to exceeding the threshold value by the measurement of the electrical conductivity of the skin of the user.

12. System (1) according to claim 10 or 11, wherein the detection means comprise at least one further sensor (61) configured to measure an ambient temperature in proximity of the user, and
wherein the software application (4) is configured to modify at least one between:
- the measurement of the electrical conductivity of the skin of the user, and
- the threshold value associated with the measurement of the electrical conductivity of the skin of the user,
when an ambient temperature above a threshold ambient temperature value or an ambient temperature outside an interval of expected ambient temperature values is measured.

13. System (1) according to any one of the preceding claims 6 to 12, wherein at least part of the detection means (60) are integrated in at least one among:
the wearable electronic device (2),
a further wearable device (3) worn by the user,
in an object used by the user during a vexing event,
a tool used by the user during the performance of said activity to be carried out, and
in a place where said activity to be carried out is to be carried out , wherein the detection means (60) comprises sensors suitable for detecting the presence of the user or of the wearable electronic device within the place.

14. Method (100; 200) for assisting the execution of programmed activities comprising the steps of:
- programming (101, 103) an activity agenda through a software application, the activity agenda comprising at least one voice message and a time instant associated with said voice message, said at least one voice message comprising an invitation to carry out an activity,
- transmitting (105) the at least one voice message and the time instant associated with the wearable electronic device worn by a user,
- reproducing (107-115), by means of the wearable electronic device, the at least one voice message at the time instant associated with the at least one voice message, **characterised in that** the method further comprises the steps of
- detecting an at least one touch of an interactive portion (13, 31) of the wearable electronic device following the reproduction of the at least one voice message,
- measuring a duration of the at least one touch,
- transmitting a number of detected touches and the measured duration of each detected touch to the software application (4), and
**in that**
- identifying a vexing event experienced by the user when it is detected at least one between:
a touch lasting longer than a threshold duration value, and
a sequence of consecutive touches within a predetermined time interval.

15. Method (100; 200) according to claim 14, further comprising the steps of:
- measuring (201) at least one physiological parameter of the user, and
- identifying (207) a vexing event in a time interval comprising the time instant associated with the at least one voice message based on said measurement of the at least one physiological parameter.

## Patentansprüche

1. System (1) zur Unterstützung der Ausführung programmierter Aktivitäten, wobei das System (1) aufweist:
- eine tragbare elektronische Vorrichtung (2), welche von einem Benutzer getragen werden kann, aufweisend ein Geräuschreproduktions-Modul (32) und einen interaktiven Abschnitt (13, 31), welches dazu ausgebildet ist, eine Berührung des Benutzers zu erkennen, und
- eine Software-Anwendung (4), welche auf einem entfernten Terminal (5) läuft, welches dazu geeignet ist, Daten mit der tragbaren elektronischen Vorrichtung (2) auszutauschen,
wobei die Software-Anwendung (4) eine Agenda (70) aufweist, welche mindestens eine Sprachnachricht abspeichert, wobei die Sprachnachricht eine Aufforderung, eine Aktivität auszuführen, aufweist und mit einem Zeitpunkt assoziiert ist, wobei die Software-Anwendung (4) dazu ausgebildet ist, die mindestens eine Sprachnachricht und den mit dieser assoziierten Zeitpunkt an die tragbare elektronische Vorrichtung (2) zu übertragen, und
wobei die tragbare elektronische Vorrichtung (2) dazu ausgerichtet ist dazu ausgebildet ist, die mindestens eine Sprachnachricht an dem mit der mindestens einen Sprachnachricht assoziierten Zeitpunkt zu reproduzieren,
**dadurch gekennzeichnet, dass**
die tragbare elektronische Vorrichtung (2) weiterhin dazu ausgebildet ist
- mindestens eine auf die Reproduktion der mindestens einen Sprachnachricht folgende Berührung des interaktiven Abschnitts (13, 31) zu erkennen,
- eine Dauer der mindestens einen Berührung zu messen,
- eine Anzahl der erkannten Berührungen und die gemessene Dauer jeder erkannten Berührung an die Software-Anwendung (4) zu übertragen, und
dadurch, dass
die Software-Anwendung (4) dazu ausgebildet ist, ein von dem Benutzer erlebtes störendes Ereignis zu identifizieren, wenn mindestens eines der folgenden erkannt wird:
eine Berührung, welche länger dauert als ein Zeitdauer-Grenzwert, und
eine Abfolge von aufeinander folgenden Berührungen innerhalb eines vorbestimmten Zeitintervalls.

2. System (1) nach Anspruch 1, wobei die Agenda (70) mindestens eine weitere Sprachnachricht, welche eine Anweisung, welche zum Abschluss der Aktivität befolgt werden soll, aufweist, abspeichert, wobei die Software-Anwendung (4) dazu ausgebildet ist, die mindestens eine weitere Sprachnachricht an die tragbare elektronische Vorrichtung (2) zu übertragen, und
Wobei die tragbare elektronische Vorrichtung (2) dazu ausgebildet ist, die mindestens eine weitere Sprachnachricht zu reproduzieren, wenn nach der Reproduktion der mindestens einen Sprachnachricht eine Berührungsdauer gleich oder kürzer als der Zeitdauer-Grenzwert erkannt wird.

3. System (1) nach Anspruch 2, wobei die tragbare elektronische Vorrichtung (2) dazu ausgebildet ist, die mindestens eine weitere Sprachnachricht nicht zu reproduzieren, wenn nach der Reproduktion der mindestens einen Sprachnachricht eine Berührung, welche länger als der Zeitdauer-Grenzwert anhält, erkannt wird.

4. System (1) nach einem der vorhergehenden Ansprüche, wobei die tragbare elektronische Vorrichtung (2) weiterhin dazu ausgebildet ist:
- eine Zeit, welche seit der Reproduktion der mindestens einen Sprachnachricht vergangen ist, zu messen,
wobei, wenn eine vorbestimmte Zeit vergangen ist, ohne dass die Berührung des Benutzers erkannt wurde, die tragbare elektronische Vorrichtung (2):
- die mindestens eine Sprachnachricht erneut reproduziert,
- eine Reproduktionszähler der mindestens einen Sprachnachricht erhöht,
- falls der Reproduktionszähler eine vorbestimmte Zahl erreicht, die mindestens eine Sprachnachricht nicht weiter reproduziert, und
- der Software-Anwendung (4) mitteilt, dass die vorbestimmte Zahl erreicht wurde, und wobei die Software-Anwendung (4) dazu ausgebildet ist, ein von dem Benutzer erlebtes störendes Ereignis zu identifizieren, wenn Information erhalten wird, dass die vorbestimmte Zahl erreicht wurde.

5. System (1) nach Anspruch 4, wobei die tragbare elektronische Vorrichtung (2) dazu ausgebildet ist:
- ein Anzahl von Ereignissen zu zählen, bei welchen:
a. die Zeit, welche zwischen der Reproduktion der mindestens eine Sprachnachricht und dem Erkennen der Berührung vergangen ist, einen Minimalwert übersteigt, und
b. die aufeinanderfolgenden Reproduktionen der Nachricht eine minimale Anzahl übersteigen, bevor die Berührung erkannt wird, und
- die Anzahl der Ereignisse der Software-Anwendung (4) mitzuteilen, und
wobei die Software-Anwendung (4) dazu ausgebildet ist die mindestens eine Sprachnachricht als nicht effektiv darzustellen.

6. System (1) nach einem der vorhergehenden Ansprüche, wobei das System (1) weiterhin Detektionsmittel (60) aufweist, welche dazu ausgebildet sind, mindestens einen physiologischen Parameter des Benutzers zu messen und die Messung des mindestens einen physiologischen Parameters an die Software-Anwendung (4) zu übertragen, und
wobei die Software-Anwendung (4) dazu ausgebildet ist, basierend auf der Messung des mindestens einen physiologischen Parameters durch die Detektionsmittel (60) ein von dem Benutzer erlebtes störendes Ereignis in einem Zeitintervall, welches den Zeitpunkt beinhaltet, zu identifizieren.

7. System (1) nach einem der vorhergehenden Ansprüche, wobei die Software-Anwendung (4) dazu ausgebildet ist, eine Alarmnachricht zu erstellen, wenn das störende Ereignis identifiziert wird.

8. System (1) nach Anspruch 2, wobei die Software-Anwendung (4) dazu ausgebildet ist:
- die mindestens eine Sprachnachricht, welche eine Aufforderung, eine Aktivität auszuführen, aufweist, aufzuzeichnen; und
- die mindestens eine weitere Sprachnachricht, welche eine Anweisung, welche zur Ausführung der Aktivität befolgt werden soll, aufweist aufzuzeichnen.

9. System (1) nach Anspruch 8, wobei die Software-Anwendung (4) dazu ausgebildet ist:
- ein Interface (70) bereitzustellen, über welches ein Bediener den mit der Sprachnachricht assoziierten Zeitpunkt auszuwählen eine Aufzeichnung der mindestens einen Sprachnachricht zu beginnen.

10. System (1) nach einem der vorhergehenden Ansprüche, wobei die Detektionsmittel (60) einen Sensor (61) aufweisen, welcher dazu ausgebildet ist, die elektrische Leitfähigkeit der Haut des Benutzers als Funktion über die Zeit zu messen, und wobei die Software-Anwendung (4) dazu ausgebildet ist, ein störendes Ereignis zu identifizieren, wenn die Leitfähigkeitsmessung einen Grenzwert übersteigt.

11. System (1) nach Anspruch 10, mindestens einen weiteren Sensor (61) aufweisen, welcher dazu ausgebildet ist, eines der folgenden zu messen:
- eine Bewegung des Benutzers,
- einen Blutvolumen-Puls des Benutzers, und
- eine Frequenz und/oder eine Varianz des Herzschlags des Benutzers, und wobei die Software-Anwendung (4) dazu ausgebildet ist, ein störendes Ereignis zu identifizieren, wenn mindestens eines der folgenden erkannt wird:
- eine wiederholende Bewegung des Benutzers,
- ein Anstieg der Umgebungstemperatur über einen Umgebungstemperatur-Grenzwert,
- einen Blutvolumen-Puls über einem Grenzwert,
- eine Abweichung einer Tendenz des Blutvolumen-Pulses von einer erwarteten Tendenz, und
- eine Frequenz und/oder eine Varianz des Herzschlags,
zusätzlich zu einer Überschreitung des Grenzwerts durch die Messung der elektrischen Leitfähigkeit der Haut des Benutzers.

12. System (1) nach einem der Ansprüche 10 oder 11, wobei die Detektionsmittel (60) mindestens einen weiteren Sensor (61) aufweisen, welcher dazu ausgebildet ist, eine Umgebungstemperatur in der Nähe des Benutzers zu messen, und die Software-Anwendung (4) dazu ausgebildet ist, mindestens eines der folgenden zu modifizieren:
- die Messung der elektrischen Leitfähigkeit der Haut des Benutzers, und
- der Grenzwert, welcher mit der Messung der elektrischen Leitfähigkeit des Benutzers assoziiert ist,
wenn eine Umgebungstemperatur über einem Umgebungstemperatur-Grenzwert oder eine Umgebungstemperatur außerhalb eines Intervalls von erwarteten Umgebungstemperatur-Werten gemessen wird.

13. System (1) nach einem der vorhergehenden Ansprüche 6 bis 12, wobei mindestens ein Teil der Detektionsmittel (60) in mindestens einem der folgenden integriert sind:
die tragbare elektronische Vorrichtung (2),
eine weitere tragbare Vorrichtung, welche von dem Benutzer getragen wird,
in einem Objekt, welches von dem Benutzer während eines störenden Ereignisses genutzt wird,
ein Werkzeug, welches von dem Benutzer während der Durchführung der auszuführenden Tätigkeit verwendet wird, und
an einem Ort, an welchem die auszuführende Tätigkeit ausgeführt werden soll, wobei die Detektionsmittel (60) Sensoren aufweisen, welche dazu geeignet sind, die Präsenz des Benutzers oder der tragbaren elektronischen Vorrichtung (2) an dem Ort zu erkennen.

14. Verfahren (100; 200) zur Unterstützung der Ausführung programmierter Aktivitäten, aufweisend die Verfahrensschritte:
- Programmieren (101, 103) einer Aktivitätsagenda durch eine Software-Anwendung, wobei die Aktivitätsagenda mindestens eine Sprachnachricht und einen mit der Sprachnachricht assoziierten Zeitpunkt abspeichert, wobei die mindestens eine Sprachnachricht eine Aufforderung, eine Aktivität auszuführen, aufweist,
- Übertragen (105) der mindestens einen Sprachnachricht und des mit der tragbaren elektronischen Vorrichtung, welche von einem Benutzer getragen wird, assoziierten Zeitpunkts,
- Reproduzieren (107-115), durch Mittel der tragbaren elektronischen Vorrichtung, der mindestens einen Sprachnachricht zu dem mit der mindestens einen Sprachnachricht assoziierten Zeitpunkt, **dadurch gekennzeichnet, dass** das Verfahren weiterhin die Verfahrensschritte aufweist:
- Erkennen mindestens einer auf die Reproduktion der mindestens einen Sprachnachricht folgenden Berührung eines interaktiven Abschnitts (13, 31) der tragbaren elektronischen Vorrichtung,
- Messen einer Dauer der mindestens einen Berührung,
- Übertragen einer Anzahl von erkannten Berührungen und der gemessen Dauer jeder erkannten Berührung an die Software-Anwendung (4),
und durch:
- Identifizieren eines von dem Benutzer erlebten störenden Ereignisses, wenn mindestens eines der folgenden erkannt wird:
eine Berührung, welche länger als ein Zeitdauer-Grenzwert anhält, und
eine Abfolge von aufeinander folgenden Berührungen innerhalb eines vorbestimmten Zeitintervalls.

15. Verfahren (100; 200) nach Anspruch 14, weiterhin aufweisend die Verfahrensschritte:
- Messen (201) mindestens eines physiologischen Parameters des Benutzers, und
- identifizieren (207) eines störenden Ereignisses in einem Zeitintervall, welches den mit der mindestens einen Sprachnachricht assoziierten Zeitpunkt beinhaltet, basierend auf der Messung des mindesten einen physiologischen Parameters des.

## Revendications

1. - Système (1) d'aide à l'exécution d'activités programmées, le système (1) comprenant:
- un dispositif électronique pouvant être porté (2), qui est apte à être porté par un utilisateur, comprenant un module de reproduction sonore (32) et une partie interactive (13, 31) configurée pour détecter un toucher de l'utilisateur, et
- une application logicielle (4) s'exécutant sur un terminal à distance (5) approprié pour échanger des données avec le dispositif électronique pouvant être porté (2),
dans lequel l'application logicielle (4) comprend un agenda (70) qui stocke au moins un message vocal, ledit message vocal comprenant une invitation à réaliser une activité et étant associé à un instant temporel, l'application logicielle (4) étant configurée pour transmettre, au dispositif électronique pouvant être porté (2), l'au moins un message vocal et l'instant temporel qui lui est associé, et
dans lequel le dispositif électronique pouvant être porté (2) est configuré pour reproduire l'au moins un message vocal à l'instant temporel associé à l'au moins un message vocal,
**caractérisé par le fait que**
le dispositif électronique pouvant être porté (2) est en outre configuré pour :
- détecter au moins un toucher de la partie interactive (13, 31) après la reproduction de l'au moins un message vocal,
- mesurer une durée de l'au moins un toucher,
- transmettre, à l'application logicielle (4), un nombre de touchers détectés et la durée mesurée de chaque toucher détecté, et
**par le fait que**
l'application logicielle (4) est configurée pour identifier un événement contrariant vécu par l'utilisateur lorsqu'il est détecté au moins l'un entre :
un toucher qui dure plus longtemps qu'une valeur de durée de seuil, et
une séquence de touchers consécutifs dans un intervalle de temps prédéterminé.

2. - Système (1) selon la revendication 1, dans lequel l'agenda (70) stocke au moins un autre message vocal contenant une instruction à suivre pour réaliser ladite activité, l'application logicielle (4) étant configurée pour transmettre ledit au moins un autre message vocal au dispositif électronique pouvant être porté, et
dans lequel le dispositif électronique pouvant être porté (2) est configuré pour reproduire ledit au moins un autre message vocal lorsqu'un toucher d'une durée inférieure ou égale à ladite durée de seuil est détecté après la reproduction de l'au moins un message vocal.

3. - Système (1) selon la revendication 2, dans lequel le dispositif électronique pouvant être porté (2) est configuré pour ne pas reproduire ledit au moins un autre message vocal lorsqu'un toucher qui dure plus longtemps que ladite durée de seuil est détecté après la reproduction de l'au moins un message vocal.

4. - Système (1) selon l'une quelconque des revendications précédentes, dans lequel le dispositif électronique pouvant être porté (2) est en outre configuré pour :
- mesurer un temps écoulé depuis la reproduction de l'au moins un message vocal, lorsqu'un temps prédéterminé s'est écoulé sans avoir détecté le toucher de l'utilisateur, le dispositif électronique pouvant être porté (2) :
- reproduit à nouveau l'au moins un message vocal,
- incrémente un compteur de reproduction de l'au moins un message vocal,
- si le compteur de reproduction atteint un nombre prédéterminé, il ne reproduit plus l'au moins un message vocal, et
- communique à l'application logicielle (4) le fait que le nombre prédéterminé a été atteint, et
dans lequel l'application logicielle (4) est configurée pour identifier un événement contrariant vécu par l'utilisateur lorsque des informations sont reçues selon lesquelles le nombre prédéterminé a été atteint.

5. - Système (1) selon la revendication 4, dans lequel le dispositif électronique pouvant être porté (2) est configuré pour :
- compter un nombre d'événements dans lesquels :
a. le temps écoulé entre la reproduction de l'au moins un message vocal et la détection de toucher dépasse une valeur minimale, et
b. les reproductions consécutives du message dépassent un nombre minimal avant la détection du toucher, et
- communiquer ledit nombre d'événements à l'application logicielle (4), et
dans lequel l'application logicielle (4) est configurée pour signaler que l'au moins un message vocal est inefficace.

6. - Système (1) selon l'une quelconque des revendications précédentes, dans lequel le système comprend en outre des moyens de détection (60) configurés pour mesurer au moins un paramètre physiologique de l'utilisateur et pour transmettre la mesure de l'au moins un paramètre physiologique à l'application logicielle (4), et
dans lequel l'application logicielle (4) est configurée pour identifier un événement contrariant vécu par l'utilisateur dans un intervalle de temps comprenant ledit instant temporel sur la base de la mesure de l'au moins un paramètre physiologique fournie par les moyens de détection (60).

7. - Système (1) selon l'une quelconque des revendications précédentes, dans lequel l'application logicielle (4) est configurée pour générer un message d'alerte lorsque l'événement contrariant est identifié.

8. - Système (1) selon la revendication 2, dans lequel l'application logicielle (4) est configurée pour :
- enregistrer l'au moins un message vocal comprenant une invitation à réaliser une activité, et
- enregistrer l'au moins un autre message vocal comprenant une instruction à suivre pour réaliser ladite activité.

9. - Système selon la revendication 8, dans lequel l'application logicielle (4) est configurée pour :
- présenter une interface (70) au moyen de laquelle un opérateur peut sélectionner l'instant temporel associé au message vocal et commencer l'enregistrement de l'au moins un message vocal.

10. - Système (1) selon l'une quelconque des revendications précédentes, dans lequel les moyens de détection (60) comprennent un capteur (61) configuré pour mesurer la conductivité électrique de la peau de l'utilisateur en fonction du temps, et
dans lequel l'application logicielle (4) est configurée pour identifier un événement contrariant lorsque la mesure de conductivité dépasse une valeur de seuil.

11. - Système (1) selon la revendication 10, dans lequel les moyens de détection comprennent au moins un autre capteur (61) configuré pour mesurer l'un parmi :
- un mouvement de l'utilisateur,
- un pouls de volume sanguin de l'utilisateur, et
- une fréquence et/ou une variabilité du rythme cardiaque de l'utilisateur, et
dans lequel l'application logicielle (4) est configurée pour identifier un événement contrariant lorsqu'il est détecté au moins l'un parmi :
- un mouvement répétitif réalisé par l'utilisateur,
- une augmentation d'une température ambiante au-dessus d'une température ambiante de seuil,
- un pouls de volume sanguin supérieur à une valeur de seuil,
- une déviation d'une tendance du pouls de volume sanguin par rapport à une tendance attendue, et
- une fréquence et/ou une variabilité du rythme cardiaque,
en plus du dépassement de la valeur de seuil par la mesure de la conductivité électrique de la peau de l'utilisateur.

12. - Système (1) selon la revendication 10 ou 11, dans lequel les moyens de détection comprennent au moins un autre capteur (61) configuré pour mesurer une température ambiante à proximité de l'utilisateur, et
dans lequel l'application logicielle (4) est configurée pour modifier au moins l'une entre:
- la mesure de la conductivité électrique de la peau de l'utilisateur, et
- la valeur de seuil associée à la mesure de la conductivité électrique de la peau de l'utilisateur,
lorsqu'une température ambiante supérieure à une valeur de température ambiante de seuil ou une température ambiante en dehors d'un intervalle de valeurs de température ambiante attendues est mesurée.

13. - Système (1) selon l'une quelconque des revendications précédentes 6 à 12, dans lequel au moins une partie des moyens de détection (60) sont intégrés dans au moins l'un parmi :
le dispositif électronique pouvant être porté (2),
un autre dispositif électronique pouvant être porté (3) porté par l'utilisateur,
dans un objet utilisé par l'utilisateur pendant un événement contrariant,
un outil utilisé par l'utilisateur pendant l'exécution de ladite activité à réaliser, et
dans un lieu où ladite activité à réaliser doit être réalisée, les moyens de détection (60) comprenant des capteurs aptes à détecter la présence de l'utilisateur ou du dispositif électronique pouvant être porté dans le lieu.

14. - Procédé (100 ; 200) d'aide à l'exécution d'activités programmées comprenant les étapes suivantes :
- programmer (101, 103) un agenda d'activités par l'intermédiaire d'une application logicielle, l'agenda d'activités comprenant au moins un message vocal et un instant temporel associé audit message vocal, ledit au moins un message vocal comprenant une invitation à réaliser une activité,
- transmettre (105) l'au moins un message vocal et l'instant temporel associé au dispositif électronique pouvant être porté qui est porté par un utilisateur,
- reproduire (107-115), au moyen du dispositif électronique pouvant être porté, l'au moins un message vocal à l'instant temporel associé à l'au moins un message vocal, **caractérisé par le fait que** le procédé comprend en outre les étapes suivantes :
- détecter au moins un toucher d'une partie interactive (13, 31) du dispositif électronique pouvant être porté après la reproduction de l'au moins un message vocal,
- mesurer une durée de l'au moins un toucher,
- transmettre, à l'application logicielle (4), un nombre de touchers détectés et la durée mesurée de chaque toucher détecté, et
**par le fait que**
- identifier un événement contrariant vécu par l'utilisateur lorsqu'il est détecté au moins l'un entre :
un toucher qui dure plus longtemps qu'une valeur de durée de seuil, et
une séquence de touchers consécutifs dans un intervalle de temps prédéterminé.

15. - Procédé (100 ; 200) selon la revendication 14, comprenant en outre les étapes suivantes :
- mesurer (201) au moins un paramètre physiologique de l'utilisateur, et
- identifier (207) un événement contrariant dans un intervalle de temps comprenant l'instant temporel associé à l'au moins un message vocal sur la base de ladite mesure de l'au moins un paramètre physiologique.
